(19) **Europäisches Patentamt / European Patent Office / Office européen des brevets**

(11) **EP 3 102 178 B1**

(12) **FASCICULE DE BREVET EUROPEEN**

(45) Date de publication et mention de la délivrance du brevet:
**01.01.2020 Bulletin 2020/01**

(21) Numéro de dépôt: **15707703.3**

(22) Date de dépôt: **06.02.2015**

(51) Int Cl.:
*A61K 8/34* (2006.01)     *A61Q 7/02* (2006.01)
*A61Q 13/00* (2006.01)    *A61P 17/14* (2006.01)

(86) Numéro de dépôt international:
**PCT/FR2015/050283**

(87) Numéro de publication internationale:
**WO 2015/118272 (13.08.2015 Gazette 2015/32)**

(54) **NOUVELLE UTILISATION DE COMPOSÉS DE PARFUMERIE CONTRE LA REPOUSSE DE POILS**

NEUARTIGE VERWENDUNG VON KÖRPERPFLEGEVERBINDUNGEN GEGEN ERNEUTES HAARWACHSTUM

NOVEL USE OF TOILETRY COMPOUNDS TO COUNTER HAIR REGROWTH

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **06.02.2014 FR 1450922**

(43) Date de publication de la demande:
**14.12.2016 Bulletin 2016/50**

(73) Titulaire: **Robertet S.A.**
**06130 Grasse (FR)**

(72) Inventeur: **PEGARD, Anthony**
**F-06130 Grasse (FR)**

(74) Mandataire: **Agasse, Stéphane et al**
**Cabinet GERMAIN & MAUREAU**
**B.P. 6153**
**69466 Lyon Cedex 06 (FR)**

(56) Documents cités:
**WO-A1-2007/115840     JP-A- H0 881 336**
**JP-A- H11 106 321      JP-A- 2003 183 693**
**US-A- 6 132 756        US-A1- 2013 018 109**
**US-A1- 2013 078 301**

- **DATABASE WPI Week 200512 2005 Thomson Scientific, London, GB; AN 2005-105287 XP002730651, & JP 2005 015408 A (KANEBO COSMETICS INC) 20 janvier 2005 (2005-01-20)**
- **DATABASE GNPD [Online] MINTEL; décembre 2012 (2012-12), "eau de parfum", XP002730652, Database accession no. 19468859**
- **DATABASE GNPD [Online] MINTEL; avril 2005 (2005-04), "2-in-1 body wash with lotion extensions", XP002730653, Database accession no. 10217581**
- **DATABASE GNPD [Online] MINTEL; juillet 2012 (2012-07), "GERANIUM BLOSSOMS & VANILLA FRAGRANCED NATURAL 4X LAUNDRY DETERGENT", XP002730654, Database accession no. 1830459**
- **SURESH V NAMPOOTHIRI ET AL: "Comparison of Essential oil Composition of Three Ginger Cultivars from Sub Himalayan Region", ASIAN PACIFIC JOURNAL OF TROPICAL BIOMEDICINE, vol. 2, no. 3, 28 décembre 2012 (2012-12-28), pages S1347-S1350, XP055143890, ISSN: 2221-1691, DOI: 10.1016/S2221-1691(12)60414-6**

**Description**

La présente invention a pour objet l'utilisation cosmétique non thérapeutique d'une combinaison synergique, d'une composition cosmétique ou d'une formulation cosmétique pour diminuer la repousse des poils chez les mammifères, de préférence après dépilation.

Chez l'homme, on cherche à supprimer ou réduire la repousse des poils sur différentes parties du corps pour des raisons essentiellement esthétiques.

Diverses procédures ont été employées pour éliminer les poils indésirables, y compris le rasage, l'électrolyse, les crèmes ou lotions dépilatoires, l'épilation à la cire, l'épilation, et les anti-androgènes thérapeutiques.

Ces procédures classiques ont généralement des inconvénients qui leur sont associés. Le rasage, par exemple, peut provoquer des coupures et des éraflures, et peut laisser la perception d'une augmentation du taux de repousse des poils. L'électrolyse, d'autre part, permet de garder une zone traitée sans poils pendant de longues périodes de temps, mais peut être coûteuse, douloureuse et laisse parfois des cicatrices. Les crèmes dépilatoires, bien que très efficaces, ne sont généralement pas recommandées pour une utilisation fréquente en raison de leur potentiel d'irritation élevé. L'épilation à la cire et la plumaison peuvent causer douleur, inconfort et une mauvaise élimination des poils de courte taille. Enfin, les anti-androgènes - qui ont été utilisés pour traiter l'hirsutisme féminin - peuvent avoir des effets secondaires indésirables. La demande de brevet JP2005-105287 décrit une composition comprenant deux alcools sesquiterpéniques (nérolidol et farnésol), utilisée pour supprimer la repousse des poils. La demande de brevet JPH0881336 décrit un extrait brut de plantes, dont le bois de santal, pour lutter contre la repousse des poils. La demande de brevet JPH11106321 décrit l'utilisation d'un extrait de plantes, dont le gingembre, pour lutter contre la repousse des poils. De façon surprenante, il a été trouvé que la combinaison synergique de quatre à sept alcools terpéniques et comprenant quatre alcools sesquiterpéniques conduit à une action synergique contre la repousse des poils notamment après dépilation.

L'invention a pour objet l'utilisation cosmétique non thérapeutique

- d'une combinaison synergique de quatre à sept alcools terpéniques et comprenant les quatre alcools sesquiterpéniques suivants : cédrénol, cédrol, nérolidol et bisabolol $\alpha$, ou
- d'une composition cosmétique comprenant 0,5 ppm de cédrénol au moins, 2 ppm de cédrol au moins, 5 ppm de nérolidol au moins et 2,5 ppm de bisabolol $\alpha$ au moins, par rapport au poids total de la composition, ladite composition comprenant au moins un autre composé, choisi notamment parmi des composés odorants, des antioxydants et/ou des solvants organiques, ou
- d'une formulation cosmétique comprenant une combinaison synergique ou une composition cosmétique telles que définies ci-dessus et comprenant en outre un excipient pharmaceutiquement et dermatologiquement acceptable

pour diminuer la repousse des poils, de préférence après dépilation.

Les alcools terpéniques de la combinaison synergique selon l'invention sont choisis parmi les alcools monoterpéniques, sesquiterpéniques ou diterpéniques ; ce sont des composés comprenant une unité d'isoprène et pouvant être classés sur la base du nombre d'unités isoprénoïdes qu'ils contiennent. Ainsi, les alcools monoterpéniques sont constitués de deux unités d'isoprène (C10), les alcools sesquiterpéniques de trois (C15) et les alcools diterpèniques de quatre (C20). Les alcools terpéniques sont notamment définis comme une classe de composés organiques présents dans les extraits utilisés en parfumerie, comme les huiles essentielles, les concrètes, les résinoides, les absolues, les extraits au CO2 supercritique ou synthétisés par voie chimique et utilisés comme ingrédients de parfums, arômes ou médicaments.

Parmi les alcools terpéniques utilisés purs ou contenus dans des extraits naturels, on trouve le cédrénol, les cédrols, le géraniol, le nérolidol, les bisabolols, le citronellol, le nérol, le terpinéol, le linalol, le menthol, le pulégol, le carveol, pinocampheol, le myrcenol, l'isopulegol, le farnesol, le lanceol, les santalols, le vetiverol, le viridiflorol, le valerianol, les tumerols, le patchoulol, l'occidol, le nootkatol, le jinkoh eremol, l'hanamyol, le guaicol, le germacradienol, le fokienol, les eudesmols, les cadinols, ou un isomère optique ou stérique de ces molécules.

La combinaison synergique selon l'invention pourra comprendre 5 % de cédrénol, 20 % de cédrol, 50 % de nérolidol et 25 % de bisabolol $\alpha$, les pourcentages étant exprimés en poids par rapport au poids total de la combinaison synergique.

Dans une autre mise en œuvre, la combinaison synergique selon l'invention comprend du cédrénol, du cédrol, du nérolidol, du bisabolol $\alpha$, du géraniol, du nérol et du citronellol.

De façon particulière, la combinaison synergique selon l'invention comprend 3,3 % de cédrénol, 13,1% de cédrol, 32,8% de nérolidol, 16,4% de bisabolol$\alpha$, 16,4% de géraniol, 16,4% de nérol et 1,6% de citronellol.

Pour l'utilisation cosmétique non thérapeutique telle que décrite précédemment, peut également être utilisée une composition cosmétique comprenant 0,5 ppm de cédrénol au moins, 2 ppm de cédrol au moins, 5 ppm de nérolidol au moins et 2,5 ppm de bisabolol $\alpha$ au moins, par rapport au poids total de la composition, ladite composition comprenant au moins un autre composé, choisi notamment parmi des composés odorants, des antioxydants et/ou des solvants organiques.

Les composés odorants sont bien connus de l'homme du métier dans le domaine de la cosmétologie. Ces composés sont présents dans une variété de classes chimiques, mais sont en général des composés volatiles et insolubles dans l'eau. Ces composés odorants se trouvent dans des extraits végétaux tels que, des huiles essentielles, des absolues, des résinoides, des extraits CO2 ou sont des produits de synthèse. Ces composés sont présents dans la composition dans une quantité suffisante pour fournir une odeur agréable qui peut être perçue par un consommateur.

Les solvants organiques susceptibles d'être utilisés sont également bien connus de l'homme du métier. On peut citer à titre d'exemples le DPG (DiPropylène Glycol), l'éthanol, le myristate d'isopropyle, le benzoyl benzoate, le triéthyl citrate ou le diethyl phtalate.

Le(s) anti-oxydant(s) susceptible(s) d'être utilisé(s) est(sont) également bien connu(s) de l'homme du métier; on peut citer à titre d'exemples le tocophérol, l'hydroxytoluène butylé (BHT), ou l'acide 3,4-dihydro-6-hydroxy-2,5,7,8-tétraméthyl-2H-1-benzopyran-2-carboxylique (Trolox®).

Dans une mise en œuvre particulière de l'invention, la composition cosmétique comprend de 400 ppm à 100% de la combinaison synergique, par rapport au poids total de la composition, de préférence de 1% à 35%.

Selon une autre mise en œuvre de l'invention, la composition cosmétique comprend au moins 30.5 % en poids de la combinaison d'alcools terpéniques contenant dans une mise en œuvre particulière 3.3 % de cedrenol, 13.1 % de cedrol, 32,8 % de nerolidol, 16,4 % de bisabolol $\alpha$, 16.4 % de geraniol, 16,4 % de nerol et 1,6 % de citronellol.

Pour l'utilisation cosmétique non thérapeutique telle que décrite précédemment, peut également être utilisée une formulation cosmétique comprenant une combinaison synergique ou une composition cosmétique telles que décrites précédemment et comprenant en outre un excipient pharmaceutiquement et dermatologiquement acceptable.

Les excipients pharmaceutiquement, dermatologiquement ou cosmétiquement acceptables sont bien connus de l'homme du métier.

La formulation cosmétique selon la présente invention peut être, par exemple, un produit cosmétique ou dermatologique, sous la forme d'une pommade, d'une lotion, d'une mousse, d'une crème, d'un gel, d'un baume, d'une solution, d'une émulsion huile dans eau ou eau dans huile, d'un onguent, d'une huile corporelle, etc.La formulation cosmétique peut aussi se présenter sous la forme d'une préparation de rasage ou d'une lotion après-rasage.

La formulation cosmétique selon la présente invention peut aussi prendre la forme d'une lotion ou d'une solution dans laquelle la composition selon l'invention est sous forme encapsulée. La composition selon l'invention peut être incorporée dans des vecteurs de type liposomes, glycosphères, cyclodextrines, dans des chylomicrons, des macro-, micro-, nano-particules ainsi que les macro-, micro- et nanocapsules et aussi être absorbés sur des polymères organiques poudreux, les talcs, bentonites et autres supports minéraux.

La formulation cosmétique selon la présente invention peut prendre la forme de gel comprenant des excipients appropriés tels que les esters de cellulose ou d'autres agents gélifiants, tels que le carbopol, le sepinov (polyacrylate), la gomme guar, etc.

La formulation cosmétique selon l'invention comprend entre 0.1% et 20% de la composition cosmétique selon l'invention.

Dans un autre aspect de l'invention, la formulation cosmétique selon l'invention comprend entre 0.4 ppm et 10%, de préférence entre 0.1% et 3%, et plus préférentiellement entre 0.1% et 1.5%, de la combinaison synergique selon l'invention, ou entre 0.1% et 10 % de la composition cosmétique selon l'invention, de préférence entre 0.1% et 3%, et plus préférentiellement entre 0.1% et 1.5%, les pourcentages étant exprimés en poids par rapport au poids total de la formulation cosmétique.

Ainsi, dans une mise en œuvre particulière de l'invention, la formulation cosmétique est un lait qui comprend 1% d'une composition cosmétique comprenant une combinaison d'alcools terpéniques selon l'invention et des solvants, par exemple le DPG, l'éthanol, le myristate d'isopropyle, le benzoyl benzoate, le triéthyl citrate ou le diethyl phtalate.

La formulation cosmétique peut éventuellement comprendre des composants qui favorisent la pénétration des alcools terpéniques. Des exemples de promoteurs de pénétration comprennent l'urée, le polyoxyéthylène (par exemple, Brij-30 et laureth-4), 3-hydroxy-3,7, 11 - triméthyl-I ,6,10-dodecatriene, acides cis-gras (par exemple, l'acide oléique, l'acide palmitoléique), l'acétone, le laurocaprame, le diméthylsulfoxide, la 2-pyrrolidone, l'alcool oléique, le glycérol-3-stéarate, le propan-2-ol, ester de l'acide myristique d'isopropyle, le cholestérol, et le propylène glycol.

Un activateur de pénétration peut être ajouté, par exemple, à des concentrations de 0,1% à 20%, de préférence de 0,5% à 5% en poids.

La formulation cosmétique selon la présente invention peut aussi contenir des additifs ou des adjuvants usuels en cosmétologie, comme par exemple des agents antimicrobiens, mais aussi des lipides d'extraction ou de synthèse, des polymères gélifiants et viscosifiants, des tensio-actifs et des émulsifiants, des principes actifs hydro- ou liposolubles, des extraits de plantes, des extraits tissulaires, des extraits marins, des actifs de synthèse.

La formulation cosmétique selon la présente invention peut aussi comprendre d'autres principes actifs complémentaires choisis pour leur action, par exemple pour l'effet amincissant, l'effet anti-cellulite, l'effet raffermissant, l'effet hydratant, l'effet anti-âge, l'effet tenseur, l'effet anti-ride, l'activité chélatante, l'activité complexante et séquestrante, l'effet anti-cernes, l'effet anti-rougeurs, l'activité émolliente, l'activité épilatoire, l'activité participant au maintien de l'ovale du visage, la nutrition cellulaire, la respiration cellulaire, l'activité anti-repousse ou la tonicité cutanée.

Lorsque la formulation cosmétique selon la présente invention contient des principes actifs complémentaires, ceux-ci sont généralement présents à une concentration suffisamment élevée pour qu'ils puissent exercer leur activité.

Les formulations cosmétiques selon la présente invention sont de préférence utilisées quotidiennement et appliquées une ou plusieurs fois par jour.

De façon particulière et selon l'un des aspects de l'invention, l'utilisation cosmétique non thérapeutique correspond à l'utilisation d'une formulation cosmétique comprenant de 0,1% à 10% de la combinaison synergique selon l'invention, de préférence de 0,1 à 3% et préférentiellement 1%, les pourcentages étant exprimés par rapport au poids total de la formulation cosmétique.

L'invention a également pour objet une méthode pour réduire la repousse des poils comprenant la sélection d'une zone de peau sur laquelle la réduction de la repousse des poils est recherchée, et l'application sur ladite zone d'une formulation cosmétique ou d'une composition cosmétique telles que décrites ci-dessus en quantité suffisante pour réduire la repousse des poils.

La zone de peau peut être notamment située sur le visage, les jambes, le pubis, le torse, les bras ou les aisselles.

Cette utilisation peut être renouvelée jusqu'à l'obtention d'un résultat acceptable par l'utilisateur.

Les formulations selon la présente invention sont très bien tolérées, elles ne présentent aucune toxicité et leur application sur la peau, pour des périodes de temps prolongées, n'implique aucun effet systémique.

La présente invention est illustrée par les exemples suivants.

***Exemple 1 : Etude de la quantité de VEGF produite par les cellules en présence d'alcool terpénique***

Principe du test

Le test repose sur l'étude de l'effet du produit testé sur des cellules de la papille dermique de follicules humains (HFDPC).

Cette étude permet d'évaluer la quantité de facteur de croissance endothélial vasculaire (VEGF alpha). Cette cytokine est surtout connue pour son activité angiogénique. Elle augmente la perméabilité vasculaire et par là, l'irrigation du tissu, avec comme conséquence, la croissance du poil (Yano et al., « Control of hair growth and follicle size by VEGF mediated angiogenesis », J. Clin. Invest., 2001, 107, p. 409-417)

Le VEGF permet, par l'augmentation de l'irrigation sanguine du tissu capillaire, une meilleure nutrition de la base du follicule. Les observations liant le VEGF à la croissance des poils sont nombreuses.

Protocole

Les cellules sont cultivées dans des plaques 12 puits. L'expérience est répétée trois fois. Une série de trois puits non traités sert de contrôle (DPG 1%). Différentes concentrations du produit à tester sont appliquées sur les cellules à confluence et laissées à incuber 24 heures.

La quantité de VEGF produit par les cellules au bout du temps d'incubation est dosée par kit ELISA (Human VEGF ELISA Development Kit, Promokine) dans le milieu de culture.

L'anticorps de capture est dilué avec du tampon PBS à une concentration de 0.5 $\mu$g/ml. Immédiatement, on ajoute 100 de cet anticorps dans chaque puits de la plaque ELISA. La plaque est scellée puis incubée toute la nuit à température ambiante.

La plaque est ensuite lavée quatre fois avec du tampon de lavage, puis 300 $\mu$l de tampon de blocage est ajouté. La plaque est mise à incuber une heure.

Des dilutions du standard de 2 ng/ml à zéro sont effectuées afin de réaliser la courbe étalon de VEGF.

Dans les autres puits, 100 $\mu$l d'échantillons sont déposés par puits, en triplicatas. La plaque est incubée deux heures à température ambiante.

Après quatre lavages, 100 $\mu$l d'anticorps de détection à 1 $\mu$g/ml sont ajoutés dans chaque puit et incubés pendant deux heures.

L'étape suivante consiste à mettre le conjugué Avidine-HRP.

Après incubation de trente minutes et lavages, 100 $\mu$l par puits de substrat ABTS sont ajoutés, et l'apparition de la couleur est mesurée à l'aide d'un lecteur de microplaques ELISA, à une longueur d'onde de 405 nm.

Résultats

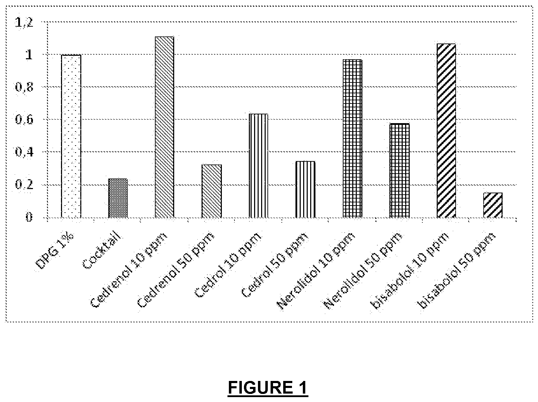

Les résultats du test sont présentés Figure 1.

Différents alcools terpéniques ont été testés seuls à différentes concentrations (Cédrénol, Cédrol, Nérolidol et Bisabol), une combinaison («cocktail») également été testée. Le « cocktail » consiste en une combinaison de néroidol, bisabolol, cédrol et cédrénol, la combinaison a été diluée de sorte que le cocktail comprend en concentration finale 5 ppm de nerolidol, 2,5 ppm de α-bisabolol, 2 ppm de cédrol et 0,5 ppm de cédrénol.

La valeur représentée en ordonnées correspond au ratio entre la quantité de VEGF sécrétée par les cellules traitées et la quantité sécrétée par les cellules « contrôles » (DPG 1%).

Les résultats présentés attestent de l'effet synergique du cocktail sur quantité de VEGF sécrétée par les cellules traitées et par conséquent sur la repousse des poils.

## *Exemple 2:*

Pour confirmer ces résultats obtenus *in vitro,* un test a été réalisé *in vivo* sur un panel de 20 personnes. Il compare l'effet d'un lait contenant la composition selon l'invention à celui d'un lait qui en est dépourvu. Les volontaires ont testé l'effet anti repousse d'un lait (formulation cosmétique) comprenant 1% d'une composition comprenant un combinaison d'alcools terpéniques (étude clinique réalisée, sur vingt volontaires, en double aveugle randomisée).

Le test a été réalisé sur 20 volontaires sur une durée de 63 jours.

Les produits testés sont les suivant :

- un placebo (625 Placébo), un lait qui comprend : une phase aqueuse (eau: 77.5%, Glycerine *(glycérine codex-AMI)* 3%), une phase grasse (Glyceryl stearate *(cutina GMS V - AMI)*: 2%, Cetearyl alcohol & cetearyl glucoside *(montanov 68E - Seppic):* 3%, Caprylic/capric triglyceride *(myritol 318 - AMI)*: 3%, Octyldodecanol *(eutanol G - AMI)*: 5%, Polysorbate 80 *(eumulgin SMO 20 - AMI)*: 1%), des conservateurs (phenoxyethanol-ethylhexylglycerin *(euxyl PE9010-Schulke)* : 1%), des épaississants (Polyacrylamide & C13-14 isoparaffin & laureth-7 (sepigel 305 - Seppic) : 1.5%) ;

- une formulation cosmétique (également notée « 749 » dans les tableaux):, le même lait que le placebo contenant 1% d'une composition cosmétique contenant 30.5% de la combinaison d'alcools terpéniques suivante : 3.3% de cedrenol, 13.2% de cedrol, 33% de nerolidol, 16% de bisabolol, 16.5% de geraniol, 16% de nerol et 2% de citronellol.

## Déroulement de l'essai

Schéma de l'essai

### *A J-21 (21 jours avant le début des tets effectifs)*

Les volontaires sont venus au laboratoire sans avoir appliqué de produit au niveau des jambes depuis la veille au soir.

Ils ont lu, signé et daté la fiche d'information (instructions sur l'utilisation du produit et restrictions relatives à l'étude) et le formulaire de consentement en double exemplaire. Ces documents sont aussi signés et datés par la personne en charge du recueil de consentement. Les volontaires en reçoivent une copie.

Le technicien responsable de l'essai vérifie les critères d'inclusion et de non-inclusion
Une esthéticienne procède à l'épilation des demi-jambes à la cire au laboratoire. Une fiche de suivi journalier est distribuée aux volontaires.

Les volontaires sont reparties chez elles avec les consignes :

- de ne pas s'épiler, ni se raser les jambes jusqu'à leur prochain rendez-vous,
- et de ne pas appliquer de produit de soin sur les jambes jusqu'au prochain rendez-vous.

### *A J0 (jour marquant le début des tests)*

Les volontaires sont venues au laboratoire sans avoir appliqué de produit de soin au niveau des jambes depuis le jour J-21 et leur dernière épilation date de la dernière visite au laboratoire (il y a 21 jours) et elles ont remis leur fiche de suivi journalier au technicien responsable de l'étude.

Une zone d'étude au niveau de chaque jambe est définie (une zone traitée et une zone non-traitée) et on procède à l'acquisition d'images au Vidéomicroscope Hirox® de chacune des zones.

Une esthéticienne procède à l'épilation des demi-jambes à la cire au laboratoire. Une fiche de suivi journalier et les produits étudiés sont distribués.

Les volontaires sont reparties chez elles avec les consignes

- de ne pas s'épiler, ni se raser les jambes jusqu'à leur prochain rendez-vous,
- et d'appliquer les produits étudiés sur les jambes (selon la randomisation prévue) deux fois par jour (matin et soir).

**A J21 et J42 (respectivement 21 et 42 jours après le début des tests)**

Les volontaires sont venues au laboratoire sans avoir appliqué de produit de soin au niveau des jambes le matin de l'étude et leur dernière épilation date de la dernière visite au laboratoire (il y a 21 jours) et elles ont remis leur fiche de suivi journalier au technicien responsable de l'étude.

Une esthéticienne procède à l'épilation des demi-jambes à la cire au laboratoire.

Les volontaires sont reparties chez elles avec les consignes

- de ne pas s'épiler, ni se raser les jambes jusqu'à leur prochain rendez-vous,
- et d'appliquer les produits étudiés sur les jambes (selon la randomisation prévue) deux fois par jour (matin et soir).

**A J63 (23 jours après le début des tests)**

Les volontaires sont venues au laboratoire sans avoir appliqué de produit de soin au niveau des jambes le matin de l'étude et leur dernière épilation date de la dernière visite au laboratoire (il y a 21 jours).

Les volontaires ont remis leur fiche de suivi journalier au technicien responsable de l'étude ainsi que les produits restant.

Les zones qui ont été définies à J-21 à l'aide du calque de repositionnement sont repérées Et on procède à l'acquisition d'images au Vidéomicroscope Hirox® de chacune des zones.

Les volontaires ont complété le questionnaire d'évaluation subjective.

Principe de l'étude :

Les zones étudiées sont visualisées à l'aide d'un vidéomicroscope. Il s'agit d'un microscope mobile à fibre optique et à objectif variable, muni d'un objectif X10, couplé à un système informatique d'acquisition d'image.

L'objectif est mis directement devant la zone étudiée, sans contact. L'image à 16 millions de couleurs, est observée sur écran informatique.

L'acquisition d'image réalisée permet de couvrir une zone d'environ 14 cm$^2$. Le traitement de cette image est réalisé par le logiciel Photoshop®.

Pour chaque étude, une calibration est effectuée à l'aide d'une image étalon (papier millimétré) qui sera utilisée pour déterminer la surface exacte étudiée.

Les paramètres évalués sont la densité pilaire (nombre total de poils par cm$^2$), la vitesse de pousse des poils (en mm par jour) et la variation de l'épaisseur des poils (entre J63 et JO) par scorage selon l'échelle suivante:

|  ❑  |  ❑  |  ❑  |
|---|---|---|
| -1 | 0 | +1 |
| poils moins épais | poils d'épaisseur égale | poils plus épais |

**Analyse des données**

**Données analysées**

Les paramètres analysés sont :

- la densité pilaire (nb/cm$^2$).
- la vitesse de pousse (en mm/jour).
- la variation de l'épaisseur des poils (par scorage).

**Formules de calcul**

Les variations brutes (A) et en pourcentages sur les moyennes (A%) des différents paramètres étudiés ont été calculées selon les formules suivantes :

$$\Delta ZT = (ZT_{ti} - ZT_{t0}) \text{ et } \Delta ZNT = (ZNT_{ti} - ZNT_{t0})$$

$$\Delta \% = \frac{(ZT_{ti} - ZT_{t0}) - (ZNT_{ti} - ZNT_{t0})}{ZT_{t0} + (ZNT_{ti} - ZNT_{t0})} \times 100$$

avec :

ZT : valeur obtenue sur la zone traitée,
ZNT : valeur obtenue sur la zone non traitée,
t0 : avant application,
ti : aux différents temps de mesures après application.

*Remarque :*

Le pourcentage de variation ($\Delta\%$) exprime en pourcentage la variation de la zone traitée ($ZT_{ti} - ZT_{t0}$) comparée à la variation de la zone non traitée ($ZNT_{ti}; - ZNT_{t0}$) :

$$(ZT_{ti} - ZT_{t0}) - (ZNT_{ti}; - ZNT_{t0})$$

Ces variations sont pondérées à la valeur initiale $ZT_{t0}$ (avant application) corrigée par la dérive possible, entre t0 et ti, indépendante du traitement. Cette dérive est évaluée sur la variation de la zone non traitée ($ZNT_{ti} - ZNT_{t0}$) :

$$ZT_{t0} + (ZNT_{ti} - ZNT_{t0})$$

Cette expression ($\Delta\%$) donne ainsi la variation en pourcentage de chaque zone traitée par rapport aux conditions initiales ($ZT_{t0}$) tout en tenant compte des fluctuations (indépendantes du traitement) de la zone non traitée.
Les valeurs mesurées sont reprises dans les tableaux de valeurs brutes. Ces tableaux présentent également les statistiques descriptives : moyennes, médianes, minima, maxima, écart-types sur la moyenne (SEM) et intervalles de confiance à 95% (IC 95%).
De même, les variations brutes, les variations en pourcentage, les statistiques descriptives et les résultats statistiques (p) sont présentés dans les tableaux de variations.

**Méthode statistique**

L'analyse statistique permet de déterminer la significativité des variations sous l'effet du produit testé.
La comparaison porte sur les valeurs obtenues sur la zone traitée et sur la zone non traitée aux différents temps d'évaluation, comparativement à avant application.
Le test utilisé est le test t de Student sur données appariées. Les conditions d'application sont le caractère aléatoire et simple des échantillons et la normalité de la population des différences.
Le principe du test consiste à poser une hypothèse nulle (H0) d'absence de différence entre l'effet moyen sur la (ou les) zone(s) traitée(s) et non traitée (d = 0) et une hypothèse alternative H1 (notre hypothèse de recherche) d'une différence entre les zones ($d<>0$).
On détermine ensuite quelle est la probabilité p d'observer un écart entre les temps au moins aussi grand que celui qui a été observé si l'hypothèse nulle est vraie.

- Si p $\leq$ 5 %, on rejette l'hypothèse nulle. On accepte alors l'hypothèse alternative H1 d'une différence significative entre les zones.

- Si p > 5%, on accepte l'hypothèse nulle. Les données n'ont pas permis de mettre en évidence une différence significative entre les zones.

**RESULTATS**

**Effet anti-repousse poils**

Les résultats individuels sont présentés dans les tableaux ci-dessous.

Les paramètres étudiés sont :

- densité pilaire (nb/zone).
- vitesse de pousse des poils (mm/ jour)
- épaisseur des poils (scores)

Une diminution d'au moins un de ces paramètres caractérise l'effet du produit sur le ralentissement de la pousse des poils.

Une synthèse des résultats est présentée ci-après.

**Evaluation de la densité pilaire**

| Variation de la densité pilaire (nbre de poils/zone) | | | | | | |
|---|---|---|---|---|---|---|
| | | | | Student-t test | | |
| Produit | Cinétique | Δ Moyenne ± SEM | Δ% | p | Significativité | %de volontaires présentant une diminution du nombre |
| 625 | Δ J63-J0 | -2,5 ± 3,3 | 8%b | 0,455 | Oui | 55% |
| 749 | Δ J63-J0 | -6,5 ± 2,1 | -19% | 0,007 | Oui | 75% |
| Comparaison des produits | Δ J63-J0 | 4,0 ± 2,9 | 15% | 0,192 | Non | |

Dans les conditions de cette étude :

- le produit "625 Placebo" a induit une diminution non significative (p=0,455) de la densité pilaire de -8% en moyenne. Une diminution a été observée chez 55% des volontaires,

- la formulation cosmétique comprenant la combinaison synergique de l'invention (le produit « 749 ») a induit une diminution significative (p=0,007) de la densité pilaire de -19% en moyenne. Une diminution a été observée chez 75% des volontaires.

**Evaluation de la vitesse de pousse des poils**

| Variation de la vitesse de pousse des poils (mm/jour) | | | | | | |
|---|---|---|---|---|---|---|
| | | | | Student-t test | | |
| | Cinétique | Δ Moyenne ± SEM | Δ % | p | Significativité | %de volontaires présentant une diminution de la vitesse de pousse des poils |
| Produit 625 | Δ J63-J0 | -0,007 ± 0,008 | -7,0% | 0,37474 | Non | 45% |
| Produit 749 | Δ J63-J0 | -0,010 ± 0,009 | -8,6% | 0,27344 | Non | 50% |
| Comparaison des produits | Δ J63-J0 | 0,002 ± 0,008 | 2,4% | 0,77338 | Non | |

Dans les conditions de cette étude, les produits "625 Placebo" et la formulation cosmétique comprenant la combinaison synergique de l'invention (« 749 ») n'ont pas induit de diminution significative de la vitesse de pousse des poils.

**Evaluation de l'épaisseur des poils**

| Variation de l'épaisseur des poils (scores) | | | | | |
|---|---|---|---|---|---|
| | | | Test de Wilcoxon | | |
| Produit | Cinétique | Δ Moyenne ± SEM | p | Significativité | %de volontaires présentant une diminution de l'épaisseur de poils |
| 625 | Δ J63-J0 | -0,2 ± 0,2 | 0,388 | Non | 40% |
| 749 | Δ J63-J0 | 0,1 ± 0,1 | 0,727 | Non | 15% |

Légende:

-1 : Poils moins épais

0 : Poils d'épaisseur identique

+1 : Poils plus épais

Dans les conditions de cette étude, les produits "625: Placebo" et la formulation cosmétique comprenant la combinaison synergique de l'invention (« 749 ») n'ont pas induit de diminution significative de l'épaisseur des poils.

**Revendications**

**1.** Utilisation cosmétique non thérapeutique

- d'une combinaison synergique de quatre à sept alcools terpéniques et comprenant les quatre alcools sesquiterpéniques suivants : cédrénol, cédrol, nérolidol et bisabolol α, ou
- d'une composition cosmétique comprenant 0,5 ppm de cédrénol au moins, 2 ppm de cédrol au moins, 5 ppm de nérolidol au moins et 2,5 ppm de bisabolol α au moins, par rapport au poids total de la composition, ladite composition comprenant au moins un autre composé, choisi notamment parmi des composés odorants, des antioxydants et/ou des solvants organiques, ou
- d'une formulation cosmétique comprenant une combinaison synergique ou une composition cosmétique telles que définies ci-dessus et comprenant en outre un excipient pharmaceutiquement et dermatologiquement acceptable

pour diminuer la repousse des poils, de préférence après dépilation.

**2.** Utilisation cosmétique non thérapeutique selon la revendication 1, dans laquelle la combinaison synergique est **caractérisée en ce que** les alcools terpéniques sont choisis parmi le géraniol, les bisabolols, le citronellol, le nérol, le terpinéol, le linalol, le menthol, le pulégol, le carveol, pinocampheol, le myrcenol, l'isopulegol, le farnesol, le lanceol, les santalols, le vetiverol, le viridiflorol, le valerianol, les tumerols, le patchoulol, l'occidol, le nootkatol, le jinkoh eremol, l'hanamyol, le guaicol, le germacradienol, le fokienol, les eudesmols, les cadinols, ou un isomère optique ou stérique de ces molécules.

**3.** Utilisation cosmétique non thérapeutique selon la revendication 1 ou 2, dans laquelle la combinaison synergique comprend 5 % de cédrénol, 20 % de cédrol, 50 % de nérolidol et 25 % de bisabolol α, les pourcentages étant exprimés en poids par rapport au poids total de la combinaison synergique.

**4.** Utilisation cosmétique non thérapeutique selon l'une des revendications 1 à 2, dans laquelle la combinaison synergique comprend du cédrénol, du cédrol, du nérolidol, du bisabolol α, du géraniol, du nérol et du citronellol.

**5.** Utilisation cosmétique non thérapeutique selon la revendication 4, dans laquelle la combinaison synergique comprend 3,3 % de cédrénol, 13,1% de cédrol, 32,8% de nérolidol, 16,4% de bisabolol α, 16,4% de géraniol, 16,4% de nérol et 1,6% de citronellol.

**6.** Utilisation cosmétique non thérapeutique selon la revendication 1, dans laquelle la formulation cosmétique comprend entre 0.4 ppm et 10%, de préférence entre 0.1% et 3%, et plus préférentiellement entre 0.1% et 1.5%, de la combinaison synergique selon l'une des revendications 1 à 5, ou entre 0.1% et 10 % de la composition cosmétique selon la revendication 1, de préférence entre 0.1% et 3%, et plus préférentiellement entre 0.1% et 1.5%, les pourcentages étant exprimés en poids par rapport au poids total de la formulation cosmétique.

**7.** Utilisation cosmétique non thérapeutique selon la revendication 1 ou 6, dans laquelle la formulation cosmétique se présente sous la forme d'une lotion, d'un gel, d'une crème, d'une pommade, d'un baume ou d'une mousse.

**8.** Utilisation cosmétique non thérapeutique selon la revendication 1, 6 ou 7, dans laquelle la formulation cosmétique comprend en outre au moins un principe actif susceptible de conférer à la formulation cosmétique des propriétés amincissante, anti-cellulite, raffermissante, hydratante, anti-âge, tenseur, anti-ride, chélatante, complexante et séquestrante, anti-cernes, anti-rougeurs, émolliente, démêlante capillaire, anti-pelliculaire, épilatoire, participant au maintien de l'ovale du visage, de nutrition cellulaire, de respiration cellulaire, anti-repousse ou de tonicité cutanée.

**Patentansprüche**

**1.** Kosmetische nicht therapeutische Verwendung

- einer synergetischen Kombination von vier bis sieben Terpenalkoholen und umfassend die vier folgenden Sesquiterpenalkohole: Cedrenol, Cedrol, Nerolidol und Bisabolol α, oder
- einer kosmetischen Zusammensetzung, umfassend mindestens 0,5 ppm Cedrenol, mindestens 2 ppm Cedrol, mindestens 5 ppm Nerolidol und mindestens 2,5 ppm Bisabolol α mit Bezug auf das Gesamtgewicht der Zusammensetzung, wobei die Zusammensetzung mindestens eine weitere Verbindung umfasst, ausgewählt insbesondere aus Duftverbindungen, Antioxidantien und/oder organischen Lösemitteln, oder
- einer kosmetischen Formulierung, umfassend eine synergetische Kombination oder eine kosmetische Zusammensetzung, wie oben definiert, und umfassend außerdem einen pharmazeutisch und dermatologisch annehmbaren Trägerstoff,

um das erneute Haarwachstum, vorzugsweise nach der Enthaarung, zu verringern.

**2.** Nicht therapeutische kosmetische Verwendung nach Anspruch 1, wobei die synergetische Kombination **dadurch gekennzeichnet ist, dass** die Terpenalkohole ausgewählt sind aus Geraniol, Bisabololen, Citronellol, Nerol, Terpineol, Linalol, Menthol, Pulegol, Carveol, Pinocampheol, Myrcenol, Isopulegol, Farnesol, Lanceol, Santalolen, Vetiverol, Viridiflorol, Valerianol, Tumerolen, Patchoulol, Occidol, Nootkatol, Jinkoheremol, Hanamyol, Guajcol, Germacradienol, Fokienol, Eudesmolen, Cadinolen oder einem optischen oder sterischem Isomer dieser Moleküle.

**3.** Nicht therapeutische kosmetische Verwendung nach Anspruch 1 oder 2, wobei die synergetische Kombination 5 % Cedrenol, 20 % Cedrol, 50 % Nerolidol und 25 % Bisabolol α umfasst, wobei die Prozentsätze in Gewicht mit Bezug auf das Gesamtgewicht der synergetischen Kombination ausgedrückt sind.

**4.** Nicht therapeutische kosmetische Verwendung nach einem der Ansprüche 1 bis 2, wobei die synergetische Kombination Cedrenol, Cedrol, Nerolidol, Bisabolol α, Geraniol, Nerol und Citronellol umfasst.

**5.** Nicht therapeutische kosmetische Verwendung nach Anspruch 4, wobei die synergetische Kombination 3,3 % Cedrenol, 13,1 % Cedrol, 32,8 % Nerolidol, 16,4% Bisabolol α, 16,4% Geraniol, 16,4% Nerol und 1,6 % Citronellol umfasst.

**6.** Nicht therapeutische kosmetische Verwendung nach Anspruch 1, wobei die kosmetische Formulierung zwischen 0,4 ppm und 10 %, vorzugsweise zwischen 0,1 % und 3 % und noch bevorzugter zwischen 0,1 % und 1,5 % der synergetischen Kombination nach einem der Ansprüche 1 bis 5 oder zwischen 0,1 % und 10 % der kosmetischen Zusammensetzung nach Anspruch 1, vorzugsweise zwischen 0,1 % und 3 %, noch bevorzugter zwischen 0,1 % und 1,5 % umfasst, wobei die Prozentsätze in Gewicht mit Bezug auf das Gesamtgewicht der kosmetischen Formulierung ausgedrückt sind.

7. Nicht therapeutische kosmetische Verwendung nach Anspruch 1 oder 6, wobei die kosmetische Formulierung die Form einer Lotion, eines Gels, einer Creme, einer Pomade, eines Balsams oder eines Schaums aufweist.

8. Nicht therapeutische kosmetische Verwendung nach Anspruch 1, 6 oder 7, wobei die kosmetische Formulierung außerdem mindestens einen Wirkstoff umfasst, der dazu in der Lage ist, der kosmetischen Formulierung schlankmachende, Anti-Cellulite-, straffende, feuchtigkeitsspendende, Anti-Aging-, spannende, Anti-Falten-, chelatbildende, komplexbildende und sequestrierende, Concealer-, Anti-Hautrötungs-, erweichende, Haarspülungs-, Antischuppen-, enthaarende-, an der Beibehaltung der Gesichtskonturen beteiligte, die Zellnutrition fördernde, die Zellatmung fördernde, das erneute Wachstum verhindernde oder die Spannkraft der Haut fördernde Eigenschaften umfasst.

## Claims

1. A non-therapeutic cosmetic use of

   - a synergistic combination of four to seven terpene alcohols and comprising the following four sesquiterpene alcohols: cedrenol, cedrol, nerolidol and bisabolol $\alpha$, or
   - a cosmetic composition comprising at least 0.5 ppm of cedrenol, at least 2 ppm of cedrol, at least 5 ppm of nerolidol and at least 2.5 ppm of bisabolol, with respect to the total weight of the composition, said composition comprising at least another compound, selected in particular from odorous compounds, antioxidants and/or organic solvents, or
   - a cosmetic formulation comprising a synergistic combination or a cosmetic composition as defined above and further comprising a pharmaceutically and dermatologically acceptable excipient

   for reducing the hair regrowth, preferably after depilation.

2. The non-therapeutic cosmetic use according to claim 1, wherein the synergistic combination is **characterized in that** the terpene alcohols are selected from geraniol, bisabolols, citronellol, nerol, terpineol, linalool, menthol, pulegol, carveol, pinocampheol, myrcenol, isopulegol, farnesol, lanceol, santalols, vetiverol, viridiflorol, valerianol, tumerols, patchoulol, occidol, nootkatol, jinkoheremol, hanamyol, guaicol, germacradienol, fokienol, eudesmols, cadinols, or an optical or steric isomer of these molecules.

3. The non-therapeutic cosmetic use according to claim 1 or 2, wherein the synergistic combination comprises 5% of cedrenol, 20% of cedrol, 50% of nerolidol and 25% of bisabolol $\alpha$, the percentages being expressed in weight with respect to the total weight of the synergistic combination.

4. The non-therapeutic cosmetic use according to any of claims 1 to 2, wherein the synergistic combination comprises cedrenol, cedrol, nerolidol, bisabolol $\alpha$, geraniol, nerol and citronellol.

5. The non-therapeutic cosmetic use according to claim 4, wherein the synergistic combination comprises 3.3% of cedrenol, 13.1% of cedrol, 32.8% of nerolidol, 16.4% of bisabolol $\alpha$, 16.4% of geraniol, 16.4% of nerol and 1.6% of citronellol.

6. The non-therapeutic cosmetic use according to claim 1, wherein the cosmetic formulation comprises between 0.4 ppm and 10%, preferably between 0.1% and 3%, and more preferably between 0.1% and 1.5%, of the synergistic combination according to any of claims 1 to 5, or between 0.1% and 10% of the cosmetic composition according to claim 1, preferably between 0.1% and 3%, and more preferably between 0.1% and 1.5%, the percentages being expressed in weight with respect to the total weight of the cosmetic formulation.

7. The non-therapeutic cosmetic use according to claim 1 or 6, wherein the cosmetic formulation is in the form of a lotion, a gel, a cream, an ointment, a balm or a foam.

8. The non-therapeutic cosmetic use according to claim 1, 6 or 7, wherein the cosmetic formulation further comprises at least one active ingredient likely to confer on the cosmetic formulation slimming, anti-cellulite, firming, moisturizing, anti-aging, tensor, anti-wrinkle, chelating, complexing and sequestering, anti-dark circles, anti-redness, emollient, hair conditioning, antidandruff, depilatory properties, participating in maintaining the oval of the face, cellular nutrition, cellular respiration, anti-regrowth or skin tonicity.

**FIGURE 1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- JP 2005105287 A
- JP H0881336 B
- JP H11106321 B

**Littérature non-brevet citée dans la description**

- **YANO et al.** Control of hair growth and follicle size by VEGF mediated angiogenesis. *J. Clin. Invest.,* 2001, vol. 107, 409-417